# EUROPEAN PATENT APPLICATION

(11) **EP 4 162 933 A1**
(43) Date of publication of application: **12.04.2023**
(21) Application number: 21201584.6
(22) Date of filing: 08.10.2021
(51) Int. Cl.: A61K 31/167, A61K 31/197, A61P 1/16

(54) **COMPOUND FOR TREATING NON-ALCOHOLIC FATTY LIVER DISEASE AND RELATED DISEASES**

(71) Applicant: Algiax Pharmaceuticals GmbH, 40699 Erkrath (DE); Deutsche Diabetes-Forschungsgesellschaft e. V., 40225 Düsseldorf (DE)
(72) Inventor: HASSE, Birgit, 40699 Erkrath (DE); KOOPMANS, Guido, 40699 Erkrath (DE); ROHBECK, Elisabeth, 40225 Düsseldorf (DE); BELGARDT, Bernd, 40225 Düsseldorf (DE); RODEN, Michael, 40225 Düsseldorf (DE); ECKEL, Jürgen, 40225 Düsseldorf (DE); ROMACHO, Tania, 40225 Düsseldorf (DE)
(74) Representative: Roth, Andy Stefan

(57) **Abstract**

Provided herein is a compound for the use in the treatment of non-alcoholic fatty liver disease (NAFLD) and related diseases such NAFL and NASH. Pharmaceutical compositions, single unit dosage forms, and kit suitable for use for the treatment of NAFLD and related diseases are also disclosed.

## Description

### FIELD OF THE DISCLOSURE

The present intervention provided herein relates to the novel use of HK4 as defined in the following in the treatment and alleviation of non-alcoholic fatty liver disease (NAFLD) and those conditions associated with NAFLD, including non-alcoholic fatty liver (NAFL) and non-alcoholic steatohepatitis (NASH) and cirrhosis.

### BACKGROUND

NAFLD is the most common liver disorder worldwide and is present in approximately 25% of the world's population. It is also very common in developed nations, such as the United States, and affected about 75 to 100 million Americans in 2017. Over 90% of obese, 60% of diabetic, and up to 20% normal-weight people develop it. NAFLD affects about 20 to 25% of people in Europe. In the United States, estimates suggest between 30 and 40% of adults have NAFLD, and about 3 to 12% of adults have NASH. The annual economic burden was approximately US$103 billion in the US in 2016.

Non-alcoholic fatty liver disease (NAFLD) encompasses a spectrum of patients who have excessive fat build-up in the liver without another clear cause such as alcohol use. There are two types: non-alcoholic fatty liver (NAFL) and non-alcoholic steatohepatitis (NASH), with the latter also including liver inflammation. NAFLD patients are often segmented into non-alcoholic fatty liver (NAFL) and non-alcoholic steatohepatitis (NASH) patients. NAFLD diagnosis requires evidence of hepatic steatosis and lack of secondary causes of liver fat accumulation such as substantial alcohol consumption, long-term use of a steatogenic medicine, or monogenic hereditary disorders. NASH is defined as the presence of >5% hepatic steatosis and inflammation with hepatocyte injury, with or without fibrosis. Although the presence of fibrosis is not required for a diagnosis of NASH, fibrosis is present in over 80% of NASH patients. For this reason, NASH patients are often further segmented by their fibrosis stage.

Non-alcoholic fatty liver disease is less dangerous than NASH and usually does not progress to NASH or liver cirrhosis. When NAFLD does progress to NASH, it may eventually lead to complications such as cirrhosis, liver cancer, liver failure, or cardiovascular disease. Non-alcoholic fatty liver disease (NAFLD) is one the fastest emerging manifestations of the metabolic syndrome worldwide. Non-alcoholic steatohepatitis (NASH) may culminate into cirrhosis and hepatocellular cancer (HCC) and is presently a leading cause of liver transplant, which creates a significant economic burden on healthcare systems, therefore augmenting the urgency to find an efficacious therapy. Although a steady progress is seen in understanding of the disease epidemiology, pathogenesis and identifying therapeutic targets, the slowest advancement is seen in the therapeutic field. Currently, there is no FDA approved therapy for this disease and appropriate therapeutic targets are urgently warranted (Raza et al. 2021).

NAFLD usually causes no signs and symptoms. The symptoms, conditions and/or symptoms associated with NAFLD include, but are not limited to, fatigue and pain or discomfort in the upper right abdomen. Possible signs and symptoms of NASH and advanced scarring (cirrhosis) include, but are not limited to, abdominal swelling (ascites), enlarged blood vessels just beneath the skin's surface, enlarged spleen, red palms and yellowing of the skin and eyes (jaundice).

NAFLD can affect both lean and obese individuals; however, the association between NAFLD and metabolic syndrome is well documented and widely recognized. It is known that obesity, type 2 diabetes mellitus (T2DM), and dyslipidemia are the most common metabolic risk factors associated with both the development and progression of NAFLD (Streba et al., 2015).

Previous studies have proposed a protective effect of GABA (gamma-Aminobutyric acid) represented in a reduced ethanol-induced cell death of rat hepatocytes (Norikura et al, 2007) or in acute liver injury (Hata et al, 2019). Liver-specific delivery of GABA-coupled nanoparticles promoted liver regeneration in partially hepatectomised rats (Shilpa et al., 2012). Furthermore, it has been reported that GABA exerts cytoprotective effects in D-galactosamine-induced liver injury in vitro and in vivo in rats (Wang et al., 2017). Taken together, available data suggest that GABA signaling may control liver cell proliferation, differentiation and exert cytoprotective effects although their precise mechanisms are poorly understood.

Initial diagnosis of NASH is based on an analysis of a patient's clinical history and a physical examination. This diagnosis focuses on potential causes, triggers, and co-morbid conditions. For an initial diagnosis to be made, five key criteria must be met: a) hepatic steatosis must be present, b) hepatocellular injury has occurred as a result of the hepatic steatosis, (c) there are no competing etiologies for the hepatic steatosis, (d) there are no co-existing causes for chronic liver disease and (e) there is no significant alcohol consumption (alcohol consumption must be within guidelines).

After a physician makes an initial diagnosis of NASH, the diagnosis may then be confirmed. Techniques used to confirm NASH diagnoses include liver biopsy, FibroScan, blood tests, and the use of non-invasive scoring scales such as the NAFLD Activity Score.

Since there is no approved drug for NASH therapy, it is crucial to look for therapeutic methods that can lead to prevention or reversal of NASH progression. It is known that high-fat, high-sugar, hypercaloric diets increase the risk of hepatic steatosis (Koopman et al., 2014). On the other hand, weight loss achieved by caloric restriction reduces hepatic inflammation and fibrosis and diminishes nonalcoholic steatohepatitis (Vilar-Gomez et al., 2015).

Another lifestyle modification important for the treatment of NAFLD that is closely related to weight loss is physical activity and exercise. It was shown that exercise significantly reduces steatosis (Keating et al., 2012) and lowers the risk of NAFLD patients progressing to NASH (Kistler et al., 2011).

Thus, no specific therapy can be recommended, and all currently prescribed drugs are used off-label. Nevertheless, there are some medicines that are already used worldwide. Among the insulin sensitizers currently available on the market, only pioglitazone was demonstrated to have some positive effects in NASH patients (improved histology and biochemistry). Although no clear statement about pioglitazone can be made (still off-label use outside T2DM), this medication could be used for NASH according to the clinical guidelines published by the joint EASL-EASD-EASO Associations. However, its usefulness for NASH is still under investigation (Pydyn et al., 2020).

Unfortunately, for many NASH patients, pharmacotherapy and lifestyle modifications are not sufficient to reduce liver fibrosis and inflammation. Since there are currently no medications directly used for the treatment of more advanced NAFLD stages, effective and improved methods and compounds that are able to treat or alleviate NAFLD and associated diseases are needed and would greatly increase the odds of reducing the global burden of NAFLD.

Against this background, it is an object underlying the present disclosure to provide compounds and methods for treating of non-alcoholic fatty liver disease and related diseases for which the disadvantages of the prior art methods can be reduced or even avoided.

### SUMMARY OF THE DISCLOSURE

In a first aspect, embodiments of this disclosure provide a compound for the use in the treatment of NAFLD and related diseases, wherein NAFLD and the related diseases are understood as above so that for further explanations it is referred to the above description.

In still another aspect, embodiments of this disclosure provide pharmaceutical compositions, single unit dosage forms, and kits suitable for use in the treatment of NAFLD and related diseases which comprises a compound according to the present disclosure.

In a further aspect, embodiments of this disclosure relate to methods of treating and preventing of NAFLD and related diseases which comprise administering to a patient in need of such treatment or prevention a therapeutically or prophylactically effective amount of a compound according to this disclosure.

Further, embodiments of this disclosure relate to HK4 or a tautomeric isoform, pharmaceutically acceptable salts, solvates, hydrates, stereoisomers, clathrates, or prodrugs thereof for use in the treatment of NAFLD and related diseases.

### BRIEF DESCRIPTION OF THE DRAWINGS

FIG. 1 shows a concentration-response curve to palmitate acid for apoptosis expressed as activity of caspase 3/7 in HepG2 liver cells.
FIG. 2 shows that HK4 treatment prevents palmitate-induced apoptosis in HepG2 liver cells and potentiates GABA.

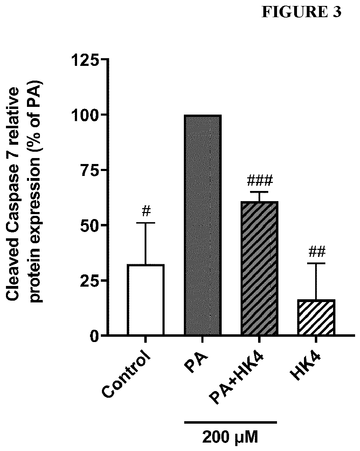

FIG. 3 shows the decrease of cleaved Caspase 7 protein levels after HK4 treatment in HepG2 cells.
FIG. 4 shows a representative human apoptosis array with marked spots for Caspase 7.
FIG. 5 shows that HK4 treatment prevents palmitate acid induced apoptosis in primary human hepatocytes.
FIG. 6 shows that HK4 prevents the palmitate-induced increase in the protein ratio of phosphorylated NF-κB to the non-phosphorylated NF-κB subunit p65.

### DETAILED DESCRIPTION OF THIS DISCLOSURE

Disclosed herein is the use of HK4 for the treatment of NAFLD and related diseases.

NAFLD according to the present disclosure is a form of fatty liver (steatosis hepatis) that is not caused by increased alcohol consumption. It includes non-alcoholic fatty liver (NAFL) and non-alcoholic steatohepatitis (NASH).

In advantageous embodiments of the present disclosure, the NAFLD and associated diseases have symptoms like fatigue and pain, discomfort in the upper right abdomen, abdominal swelling (ascites), enlarged blood vessels just beneath the skin's surface, enlarged spleen, red palms, yellowing of the skin and eyes (jaundice).

In an advantageous embodiment according to the present invention the NAFLD and associated diseases is caused by excessive fat overload in the liver and liver inflammation.

In advantageous embodiments, the compound used in the treatment of NAFLD according to the present disclosure are administered to the patient after a damage of the liver. Preferably, HK4 is administered to the patient after a damage of the liver.

The specific compound of the disclosure is described in U.S: patent nos. 5,532,259, in the international patent application WO 91/717748 which is incorporated herein by reference.

In U.S: patent nos. 5,532,259 an example for the preparation of the compound according to the present description is shown, which is incorporated herein by reference.

In advantageous embodiments, the compound is HK4 or a tautomeric isoform, a pharmaceutically acceptable salt, solvate or stereoisomer thereof.

A specific example of the compound used for the treatment of NAFLD and related diseases, has the following structure (formula I):

### I) HK4

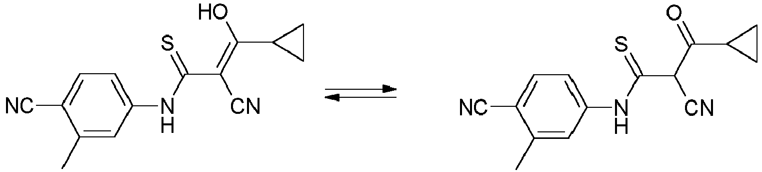

In further advantageous embodiments, the compound is 2-Cyano-N-(4-cyano-3-methyl-phenyl)-3-cyclopropyl-3-hydroxy-thioacrylamide or a tautomeric isoform, a pharmaceutically acceptable salt, solvate or stereoisomer thereof.

In further advantageous embodiments, the compound according to the present disclosure is used as the only physically active compound in the treatment of NAFLD and related diseases without a second active agent.

In yet other advantageous embodiments, the disclosure relates to pharmaceutical compositions for alleviating and/or treating NAFLD and related diseases, which comprises a therapeutically effective amount of the compound according to the present disclosure in admixture with a pharmaceutical acceptable carrier or excipient.

In advantageous embodiments, the pharmaceutical composition according to the present disclosure comprises a compound according to the present disclosure and no second active ingredient in the composition.

In an advanced embodiment, HK4 is used as the sole active agent for the treatment of NAFLD and related diseases.

In advantageous embodiments, the pharmaceutical composition is used for preventing and/or treating NAFLD and related diseases, whereby the composition comprises a therapeutically effective amount of HK4 or a physiologically functional derivative thereof in admixture with a pharmaceutical acceptable carrier or excipient.

In advantageous embodiments the pharmaceutical composition comprises 2-Cyano-N-(4-cyano-3-methyl-phenyl)-3-cyclopropyl-3-hydroxy-thioacrylamide or a tautomeric isoform, a pharmaceutically acceptable salt, solvate or stereoisomer thereof.

The compound according to the disclosure can either be commercially purchased or prepared according to the methods described in the patents or patent publications disclosed herein. Further, optically pure compositions can be asymmetrically synthesized or resolved using known resolving agents or chiral columns as well as other standard synthetic organic chemistry techniques. The compound used in the disclosure may include compounds that are racemic, stereomerically enriched or stereomerically pure, and pharmaceutically acceptable salts, solvates, stereoisomers, and prodrugs thereof.

Preferred compound used according to the disclosure is a small organic molecule having a molecular weight less than about 1,000 g/mol, and are not proteins, peptides, oligonucleotides, oligosaccharides, or other macromolecules.

As used herein and unless otherwise indicated, the term "pharmaceutically acceptable salt" encompasses non-toxic acid and base addition salts of the compound to which the term refers. Acceptable non-toxic acid addition salts include those derived from organic and inorganic acids or bases know in the art, which include, for example, hydrochloric acid, hydrobromic acid, phosphoric acid, sulfuric acid, methanesulphonic acid, acetic acid, tartaric acid, lactic acid, succinic acid, citric acid, malic acid, maleic acid, sorbic acid, aconitic acid, salicylic acid, phthalic acid, embolic acid, enanthic acid, and the like. Compounds that are acidic in nature are capable of forming salts with various pharmaceutically acceptable bases. The bases that can be used to prepare pharmaceutically acceptable base addition salts of such acidic compounds are those that form non-toxic base addition salts, i.e., salts containing pharmacologically acceptable cations such as, but not limited to, alkali metal or alkaline earth metal salts and the calcium, magnesium, sodium or potassium salts in particular. Suitable organic bases include, but are not limited to N,N-dibenzylethylenediamine, chloroprocaine, choline, diethanolamine, ethylenediamine, meglumaine (N-methylglucamine), lysine, and procaine.

As used herein, and unless otherwise specified, the term "solvate" means the compound of the present disclosure or a salt thereof that further includes a stoichiometric or non-stoichiometric amount of solvent bound by non-covalent intermolecular forces. Where the solvent is water, the solvate is a hydrate.

As used herein and unless otherwise indicated, the term "prodrug" means a derivative of a compound that can hydrolyze, oxidize, or otherwise react under biological conditions (*in vitro or in vivo*) to provide the compound. Examples of prodrugs include, but are not limited to, derivatives of the compound according to the present disclosure that comprise biohydrolyzable moieties such as biohydrolyzable amides, biohydrolyzable esters, biohydrolyzable carbamates, biohydrolyzable carbonates, biohydrolyzable ureides, and biohydrolyzable phosphate analogues. Other examples of prodrugs include derivatives of immunomodulatory compounds of the disclosure that comprise -NO, -NO2, -ONO, or - ONO2 moieties. Prodrugs can typically be prepared using well-known methods, such as those described in Burger's Medicinal Chemistry and Drug Discovery, 172-178, 949-982 (Manfred E. Wolff ed., 5th ed. 1995), and Design of Prodrugs (H. Bundgaard ed., Elselvier, New York 1985). As used herein and unless otherwise indicated, the terms "biohydrolyzable amide," "biohydrolyzable ester," "biohydrolyzable carbamate," "biohydrolyzable carbonate," "biohydrolyzable ureide," "biohydrolyzable phosphate" mean an amide, ester, carbamate, carbonate, ureide, or phosphate, respectively, of a compound that either: 1) does not interfere with the biological activity of the compound but can confer upon that compound advantageous properties in vivo, such as uptake, duration of action, or onset of action; or 2) is biologically inactive but is converted in vivo to the biologically active compound. Examples of biohydrolyzable esters include, but are not limited to, lower alkyl esters, lower acyloxyalkyl esters (such as acetoxylmethyl, acetoxyethyl, aminocarbonyloxymethyl, pivaloyloxymethyl, and pivaloyloxyethyl esters), lactonyl esters (such as phthalidyl and thiophthalidyl esters), lower alkoxyacyloxyalkyl esters (such as methoxycarbonyl- oxymethyl, ethoxycarbonyloxyethyl and isopropoxycarbonyloxyethyl esters), alkoxyalkyl esters, choline esters, and acylamino alkyl esters (such as acetamidomethyl esters). Examples of biohydrolyzable amides include, but are not limited to, lower alkyl amides, [alpha]-amino acid amides, alkoxyacyl amides, and alkylaminoalkylcarbonyl amides. Examples of biohydrolyzable carbamates include, but are not limited to, lower alkylamines, substituted ethylenediamines, amino acids, hydroxyalkylamines, heterocyclic and heteroaromatic amines, and polyether amines.

As used herein, and unless otherwise specified, the term "stereoisomer" encompasses all enantiomerically/stereomerically pure and enantiomerically/ stereomerically enriched compounds of this disclosure.

As used herein, and unless otherwise indicated, the term "stereomerically pure" or "enantiomerically pure" means that a compound comprises one stereoisomer and is substantially free of its counter stereoisomer or enantiomer. For example, a compound is stereomerically or enantiomerically pure when the compound contains 80%, 90%, or 95% or more of one stereoisomer and 20%, 10%, or 5% or less of the counter stereoisomer, in certain cases, a compound of the disclosure is considered optically active or stereomerically/enantiomerically pure {i.e., substantially the R-form or substantially the S-form) with respect to a chiral center when the compound is about 80% ee (enantiomeric excess) or greater, preferably, equal to or greater than 90% ee with respect to a particular chiral center, and more preferably 95% ee with respect to a particular chiral center.

As used herein, and unless otherwise indicated, the term "stereomerically enriched" or "enantiomerically enriched" encompasses racemic mixtures as well as other mixtures of stereoisomers of compounds of this disclosure {e.g., R/S = 30/70, 35/65, 40/60, 45/55, 55/45, 60/40, 65/35 and 70/30). Various inhibitor compounds of the present disclosure contain one or more chiral centers and can exist as racemic mixtures of enantiomers or mixtures of diastereomers. This disclosure encompasses the use of stereomerically pure forms of such compounds, as well as the use of mixtures of those forms. For example, mixtures comprising equal or unequal amounts of the enantiomers of a particular inhibitor compound of the disclosure may be used in methods and compositions of the disclosure. These isomers may be asymmetrically synthesized or resolved using standard techniques such as chiral columns or chiral resolving agents. See, e.g., Jacques, J., et al, Enantiomers, Racemates and Resolutions (Wiley-Interscience, New York, 1981); Wilen, S. H., et al, Tetrahedron 33:2725 (1977); Eliel, E. L., Stereochemistry of Carbon Compounds (McGraw-Hill, NY, 1962); and Wilen, S. H., Tables of Resolving Agents and Optical Resolutions p. 268 (E.L. Eliel, Ed., Univ. of Notre Dame Press, Notre Dame, IN, 1972).

It should be noted that if there is a discrepancy between a depicted structure and a name given that structure, the depicted structure is to be accorded more weight. In addition, if the stereochemistry of a structure or a portion of a structure is not indicated with, for example, bold or dashed lines, the structure or portion of the structure is to be interpreted as encompassing all stereoisomers of it.

The term "physiologically functional derivative" as used herein refers to compounds which are not pharmaceutically active themselves, but which are transformed into their pharmaceutical active form in vivo, i. e. in the subject to which the compound is administered. Examples of physiologically functional derivatives are prodrugs such as those described below in the present application.

The term" derivative" as used herein refers to a compound that is derived from a similar compound or a compound that can be imagined to arise from another compound, if one atom is replaced with another atom or group of atoms. The term" derivative" as used herein refers also to a compound that at least theoretically can be formed from the precursor compound (see Oxford Dictionary of Biochemistry and Molecular Biology. Oxford University Press. ISBN 0-19-850673-2.) In advantageous embodiments of the present disclosure the term "derivative" is used for derivatives from HK4.

The disclosure is also directed to the use of compound of the formula I and of its tautomeric isoform, its pharmacologically tolerable salt or physiologically functional derivative for the production of a medicament for the prevention and treatment of NAFLD and related disease.

Methods and uses according to the present disclosure encompass methods of preventing, treating and/or managing NAFLD and related diseases, but are not limited to, NAFLD, NASH and NAFL

NAFLD usually causes no signs and symptoms. The symptoms, conditions and/or symptoms associated with NAFLD include, but are not limited to, fatigue and pain or discomfort in the upper right abdomen. Possible signs and symptoms of NASH and advanced scarring (cirrhosis) include, but are not limited to, abdominal swelling (ascites), enlarged blood vessels just beneath the skin's surface, enlarged spleen, red palms and yellowing of the skin and eyes (jaundice).

The suitability of a particular route of administration of a compound according to the present disclosure employed for a particular active agent will depend on the active agent itself (e.g., whether it can be administered orally without decomposing prior to entering the blood stream) and the disease being treated. An advantageous embodiment of the route of administration for a compound according to the present disclosure is orally. Further routes of administration are known to those of ordinary skill in the art.

The dosage of therapeutically effective amount of the compound varies from and also depends upon the age and condition of each individual patient to be treated. In an embodiment of the present disclosure, the recommended daily dose range of a compound according to the present disclosure for the conditions and disorders described herein lies within the range of from about, a daily dose of about 1 mg-10g/body, preferable 5 mg-5g/body and more preferable 10 mg-2g/body of the active ingredient is generally given for preventing and /or treating this disease, and an average single dose of about 0.5-1 mg, 5 mg, 10 mg, 50 mg, 100 mg, 250 mg, 500 mg, 1 g, 2 g and 3 g is generally administered. Daily dose for administration in humans for preventing this disease (NAFLD and related diseases) could be in the range of about 0.1-50 mg/kg, wherein in particular "body" means an animal or human body.

While the term for administering of the compound to prevent this disease (NAFLD and related diseases) varies depending on species, and the nature and severity of the condition to be prevented, the compound may usually be administered to humans for a short term or a long term, i.e. for 1 week to 1 year.

Pharmaceutical compositions can be used in the preparation of individual, single unit dosage forms. The compound of the present disclosure can be used in the form of pharmaceuticals compositions, for example, in solid, semisolid or liquid form, which contains the compound according to the present disclosure as active ingredient associated with pharmaceutically acceptable carriers or excipient suitable for oral, parenteral such as intravenous, intramuscular, intrathecal, subcutaneous, enteral, intrarectal or intranasal administration. The active ingredient may be compounded, for example, with the usual non-toxic, pharmaceutically acceptable carriers for tablets, pellets, capsules, suppositories, solutions (saline for example), emulsion, suspensions (olive oil, for example), ointment and any other form suitable for use. The carriers which can be used are water, glucose, lactose gum acacia, gelatine, manitol, starch paste, magnesium trisilicate, corn starch, keratin, colloidal silica, potato starch, urea and other carriers suitable for use in manufacturing preparations, in solid, semisolid or liquid form, and in addition auxiliary, stabilizing, thickening and colouring agents and perfumes may be used. The active object compound is included in the pharmaceutical composition in an effective amount sufficient to prevent and/or treat the disease.

Single unit dosage forms of the disclosure are suitable for oral, mucosal (e.g., nasal, sublingual, vaginal, buccal, or rectal), parenteral (e.g., subcutaneous, intravenous, bolus injection, intramuscular, or intraarterial), topical (e.g., eye drops or other ophthalmic preparations), transdermal or transcutaneous administration to a patient. Examples of dosage forms include, but are not limited to: tablets; caplets; capsules, such as soft elastic gelatin capsules; cachets; troches; lozenges; dispersions; suppositories; powders; aerosols (e.g., nasal sprays or inhalers); gels; liquid dosage forms suitable for oral or mucosal administration to a patient, including suspensions (e.g., aqueous or non-aqueous liquid suspensions, oil-in- water emulsions, or a water-in-oil liquid emulsions), solutions, and elixirs; liquid dosage forms suitable for parenteral administration to a patient; eye drops or other ophthalmic preparations suitable for topical administration; and sterile solids (e.g., crystalline or amorphous solids) that can be reconstituted to provide liquid dosage forms suitable for parenteral administration to a patient.

The composition, shape, and type of dosage forms of the disclosure will typically vary depending on their use. For example, a dosage form used in the acute treatment of a disease may contain larger amounts of the active agent it comprises than a dosage form used in the chronic treatment of the same disease. Similarly, a parenteral dosage form may contain smaller amounts of the active agent it comprises than an oral dosage form used to treat the same disease. These and other ways in which specific dosage forms encompassed by this disclosure will vary from one another will be readily apparent to those skilled in the art. See, e.g., Remington's Pharmaceutical Sciences, 18th ed., Mack Publishing, Easton PA (1990).

Typical pharmaceutical compositions and dosage forms comprise one or more excipients. Suitable excipients are well known to those skilled in the art of pharmacy, and non-limiting examples of suitable excipients are provided herein. Whether a particular excipient is suitable for incorporation into a pharmaceutical composition or dosage form depends on a variety of factors well known in the art including, but not limited to, the way in which the dosage form will be administered to a patient. For example, oral dosage forms such as tablets may contain excipients not suited for use in parenteral dosage forms. The suitability of a particular excipient may also depend on the specific active agents in the dosage form. For example, the decomposition of some active agents may be accelerated by some excipients such as lactose, or when exposed to water. Active agents that comprise primary or secondary amines are particularly susceptible to such accelerated decomposition. Consequently, this disclosure encompasses pharmaceutical compositions and dosage forms that contain little, if any, lactose or other mono- or di-saccharides. As used herein, the term "lactose-free" means that the amount of lactose present, if any, is insufficient to substantially increase the degradation rate of an active ingredient.

Lactose-free compositions of the disclosure can comprise excipients that are well known in the art and are listed, for example, in the U.S. Pharmacopeia (USP) 25-NF20 (2002). In general, lactose-free compositions comprise active ingredients, a binder/filler, and a lubricant in pharmaceutically compatible and pharmaceutically acceptable amounts. Preferred lactose-free dosage forms comprise active ingredients, microcrystalline cellulose, pre-gelatinized starch, and magnesium stearate.

This disclosure further encompasses anhydrous pharmaceutical compositions and dosage forms comprising active ingredients, since water can facilitate the degradation of some compounds. For example, the addition of water (e.g., 5%) is widely accepted in the pharmaceutical arts as a means of simulating long-term storage in order to determine characteristics such as shelf-life or the stability of formulations over time. See, e.g., Jens T. Carstensen, Drug Stability: Principles & Practice, 2d. Ed., Marcel Dekker, NY, NY, 1995, pp. 379-80. In effect, water and heat accelerate the decomposition of some compounds. Thus, the effect of water on a formulation can be of great significance since moisture and/or humidity are commonly encountered during manufacture, handling, packaging, storage, shipment, and use of formulations.

Anhydrous pharmaceutical compositions and dosage forms of the disclosure can be prepared using anhydrous or low moisture containing ingredients and low moisture or low humidity conditions. Pharmaceutical compositions and dosage forms that comprise lactose and at least one active ingredient that comprise a primary or secondary amine are preferably anhydrous if substantial contact with moisture and/or humidity during manufacturing, packaging, and/or storage is expected. An anhydrous pharmaceutical composition should be prepared and stored such that its anhydrous nature is maintained. Accordingly, anhydrous compositions are preferably packaged using materials known to prevent exposure to water such that they can be included in suitable formulary kits. Examples of suitable packaging include, but are not limited to, hermetically sealed foils, plastics, unit dose containers (e.g. vials), blister packs, and strip packs.

The disclosure further encompasses pharmaceutical compositions and dosage forms that comprise one or more compounds that reduce the rate by which an active ingredient will decompose. Such compounds, which are referred to herein as "stabilizers," include, but are not limited to, antioxidants such as ascorbic acid, pH buffers, or salt buffers.

Like the amounts and types of excipients, the amounts and specific types of active agents in a dosage form may differ depending on factors such as, but not limited to, the route by which it is to be administered to patients. However, typical dosage forms of the disclosure comprise a compound according to the present disclosure or a pharmaceutically acceptable salt, solvate, hydrate, stereoisomer, clathrate, or prodrug thereof in an amount of from about 0.10 to about 150 mg. Typical dosage forms comprise a compound according to the present disclosure or a pharmaceutically acceptable salt, solvate, hydrate, stereoisomer, clathrate, or prodrug thereof in an amount of about 0.1, 1, 2, 5, 7.5, 10, 12.5, 15, 17.5, 20, 25, 50, 100, 150 or 200 mg. In a particular embodiment, a preferred dosage form comprises 4-(amino)-2-(2,6- dioxo(3-piperidyl))-isoindoline-1,3-dione in an amount of about 1, 2, 5, 10, 25 or 50mg. In a specific embodiment, a preferred dosage form comprises 3-(4-amino-1-oxo-1,3-dihydro- isoindol-2-yl)-piperidine-2,6-dione in an amount of about 5, 10, 25 or 50mg.

Oral Dosage Forms of pharmaceutical compositions of the disclosure that are suitable for oral administration can be presented as discrete dosage forms, such as, but are not limited to, tablets (e.g., chewable tablets), caplets, capsules, and liquids (e.g., flavored syrups). Such dosage forms contain predetermined amounts of active ingredient, and may be prepared by methods of pharmacy well known to those skilled in the art. See generally, Remington's Pharmaceutical Sciences, 18th ed., Mack Publishing, Easton PA (1990).

Typical oral dosage forms of the disclosure are prepared by combining the active ingredients in an intimate admixture with at least one excipient according to conventional pharmaceutical compounding techniques. Excipients can take a wide variety of forms depending on the form of preparation desired for administration. For example, excipients suitable for use in oral liquid or aerosol dosage forms include, but are not limited to, water, glycols, oils, alcohols, flavoring agents, preservatives, and coloring agents. Examples of excipients suitable for use in solid oral dosage forms {e.g., powders, tablets, capsules, and caplets) include, but are not limited to, starches, sugars, micro-crystalline cellulose, diluents, granulating agents, lubricants, binders, and disintegrating agents.

Because of their ease of administration, tablets and capsules represent the most advantageous oral dosage unit forms, in which case solid excipients are employed. If desired, tablets can be coated by standard aqueous or nonaqueous techniques. Such dosage forms can be prepared by any of the methods of pharmacy. In general, pharmaceutical compositions and dosage forms are prepared by uniformly and intimately admixing the active ingredients with liquid carriers, finely divided solid carriers, or both, and then shaping the product into the desired presentation if necessary.

For example, a tablet can be prepared by compression or molding. Compressed tablets can be prepared by compressing in a suitable machine the active ingredients in a free-flowing form such as powder or granules, optionally mixed with an excipient. Molded tablets can be made by molding in a suitable machine a mixture of the powdered compound moistened with an inert liquid diluent.

Examples of excipients that can be used in oral dosage forms of the disclosure include, but are not limited to, binders, fillers, disintegrants, and lubricants. Binders suitable for use in pharmaceutical compositions and dosage forms include, but are not limited to, corn starch, potato starch, or other starches, gelatin, natural and synthetic gums such as acacia, sodium alginate, alginic acid, other alginates, powdered tragacanth, guar gum, cellulose and its derivatives {e.g., ethyl cellulose, cellulose acetate, carboxymethyl cellulose calcium, sodium carboxymethyl cellulose), polyvinyl pyrrolidone, methyl cellulose, pre-gelatinized starch, hydroxypropyl methyl cellulose, {e.g., Nos. 2208, 2906, 2910), microcrystalline cellulose, and mixtures thereof.

Suitable forms of microcrystalline cellulose include, but are not limited to, the materials sold as AVICEL-PH-101, AVICEL-PH-103 AVICEL RC-581, AVICEL-PH-105 (available from FMC Corporation, American Viscose Division, Avicel Sales, Marcus Hook, PA), and mixtures thereof. A specific binder is a mixture of microcrystalline cellulose and sodium carboxymethyl cellulose sold as AVICEL RC-581. Suitable anhydrous or low moisture excipients or additives include AVICEL-PH- 103(TM) and Starch 1500 LM. Examples of fillers suitable for use in the pharmaceutical compositions and dosage forms disclosed herein include, but are not limited to, talc, calcium carbonate (e.g., granules or powder), microcrystalline cellulose, powdered cellulose, dextrates, kaolin, mannitol, silicic acid, sorbitol, starch, pre-gelatinized starch, and mixtures thereof. The binder or filler in pharmaceutical compositions of the disclosure is typically present in from about 50 to about 99 weight percent of the pharmaceutical composition or dosage form.

Disintegrants are used in the compositions of the disclosure to provide tablets that disintegrate when exposed to an aqueous environment. Tablets that contain too much disintegrant may disintegrate in storage, while those that contain too little may not disintegrate at a desired rate or under the desired conditions. Thus, a sufficient amount of disintegrant that is neither too much nor too little to detrimentally alter the release of the active ingredients should be used to form solid oral dosage forms of the disclosure. The amount of disintegrant used varies based upon the type of formulation and is readily discernible to those of ordinary skill in the art. Typical pharmaceutical compositions comprise from about 0.5 to about 15 weight percent of disintegrant, preferably from about 1 to about 5 weight percent of disintegrant.

Disintegrants that can be used in pharmaceutical compositions and dosage forms of the disclosure include, but are not limited to, agar-agar, alginic acid, calcium carbonate, microcrystalline cellulose, croscarmellose sodium, crospovidone, polacrilin potassium, sodium starch glycolate, potato or tapioca starch, other starches, pre-gelatinized starch, other starches, clays, other algins, other celluloses, gums, and mixtures thereof.

Lubricants that can be used in pharmaceutical compositions and dosage forms of the disclosure include, but are not limited to, calcium stearate, magnesium stearate, mineral oil, light mineral oil, glycerin, sorbitol, mannitol, polyethylene glycol, other glycols, stearic acid, sodium lauryl sulfate, talc, hydrogenated vegetable oil (e.g., peanut oil, cottonseed oil, sunflower oil, sesame oil, olive oil, corn oil, and soybean oil), zinc stearate, ethyl oleate, ethyl laureate, agar, and mixtures thereof. Additional lubricants include, for example, a syloid silica gel (AEROSIL200, manufactured by W.R. Grace Co. of Baltimore, MD), a coagulated aerosol of synthetic silica (marketed by Degussa Co. of Piano, TX), CAB-O-SIL (a pyrogenic silicon dioxide product sold by Cabot Co. of Boston, MA), and mixtures thereof. If used at all, lubricants are typically used in an amount of less than about 1 weight percent of the pharmaceutical compositions or dosage forms into which they are incorporated.

A preferred solid oral dosage form of the disclosure comprises the compound of the disclosure, anhydrous lactose, microcrystalline cellulose, polyvinylpyrrolidone, stearic acid, colloidal anhydrous silica, and gelatin.

The active ingredient of the disclosure can be administered by controlled release means or by delivery devices that are well known to those of ordinary skill in the art. Examples include, but are not limited to, those described in U.S. Patent Nos.: 3,845,770; 3,916,899; 3,536,809; 3,598,123; and 4,008,719, 5,674,533, 5,059,595, 5,591,767, 5,120,548, 5,073,543, 5,639,476, 5,354,556, and 5,733,566, each of which is incorporated herein by reference. Such dosage forms can be used to provide slow or controlled-release of one or more active ingredients using, for example, hydropropylmethyl cellulose, other polymer matrices, gels, permeable membranes, osmotic systems, multilayer coatings, microparticles, liposomes, microspheres, or a combination thereof to provide the desired release profile in varying proportions. Suitable controlled-release formulations known to those of ordinary skill in the art, including those described herein can be readily selected for use with the active ingredient of the disclosure. The disclosure thus encompasses single unit dosage forms suitable for oral administration such as, but not limited to, tablets, capsules, gelcaps, and caplets that are adapted for controlled-release.

All controlled-release pharmaceutical products have a common goal of improving drug therapy over that achieved by their non-controlled counterparts. Ideally, the use of an optimally designed controlled-release preparation in medical treatment is characterized by a minimum of drug substance being employed to cure or control the condition in a minimum amount of time. Advantages of controlled-release formulations include extended activity of the drug, reduced dosage frequency, and increased patient compliance. In addition, controlled-release formulations can be used to affect the time of onset of action or other characteristics, such as blood levels of the drug, and can thus affect the occurrence of side (e.g., adverse) effects.

Most controlled-release formulations are designed to initially release an amount of drug (active ingredient) that promptly produces the desired therapeutic effect, and gradually and continually release of other amounts of drug to maintain this level of therapeutic or prophylactic effect over an extended period of time. In order to maintain this constant level of drug in the body, the drug must be released from the dosage form at a rate that will replace the amount of drug being metabolized and excreted from the body. Controlled- release of an active ingredient can be stimulated by various conditions including, but not limited to, pH, temperature, enzymes, water, or other physiological conditions or compounds.

Parenteral dosage forms can be administered to patients by various routes including, but not limited to, subcutaneous, intravenous (including bolus injection), intramuscular, and intraarterial. Because their administration typically bypasses patients' natural defences against contaminants, parenteral dosage forms are preferably sterile or capable of being sterilized prior to administration to a patient. Examples of parenteral dosage forms include, but are not limited to, solutions ready for injection, dry products ready to be dissolved or suspended in a pharmaceutically acceptable vehicle for injection, suspensions ready for injection, and emulsions. Suitable vehicles that can be used to provide parenteral dosage forms of the disclosure are well known to those skilled in the art. Examples include, but are not limited to: Water for Injection USP; aqueous vehicles such as, but not limited to, Sodium Chloride Injection, Ringer's Injection, Dextrose Injection, Dextrose and Sodium Chloride Injection, and Lactated Ringer's Injection; water-miscible vehicles such as, but not limited to, ethyl alcohol, polyethylene glycol, and polypropylene glycol; and non-aqueous vehicles such as, but not limited to, corn oil, cottonseed oil, peanut oil, sesame oil, ethyl oleate, isopropyl myristate, and benzyl benzoate.

Compounds that increase the solubility of the active ingredient disclosed herein can also be incorporated into the parenteral dosage forms of the disclosure. For example, cyclodextrin and its derivatives can be used to increase the solubility of a compound of the disclosure and its derivatives. See, e.g., U.S. Patent No. 5,134,127, which is incorporated herein by reference.

Topical and mucosal dosage forms of the disclosure include, but are not limited to, sprays, aerosols, solutions, emulsions, suspensions, eye drops or other ophthalmic preparations, or other forms known to one of skill in the art. See, e.g., Remington's Pharmaceutical Sciences, 16th and 18th eds., Mack Publishing, Easton PA (1980 & 1990); and Introduction to Pharmaceutical Dosage Forms, 4th ed., Lea & Febiger, Philadelphia (1985). Dosage forms suitable for treating mucosal tissues within the oral cavity can be formulated as mouthwashes or as oral gels.

Suitable excipients {e.g. carriers and diluents) and other materials that can be used to provide topical and mucosal dosage forms encompassed by this disclosure are well known to those skilled in the pharmaceutical arts, and depend on the particular tissue to which a given pharmaceutical composition or dosage form will be applied. With that fact in mind, typical excipients include, but are not limited to, water, acetone, ethanol, ethylene glycol, propylene glycol, butane-1,3-diol, isopropyl myristate, isopropyl palmitate, mineral oil, and mixtures thereof to form solutions, emulsions or gels, which are non-toxic and pharmaceutically acceptable. Moisturizers or humectants can also be added to pharmaceutical compositions and dosage forms if desired. Examples of such additional ingredients are well known in the art. See, e.g., Remington 's Pharmaceutical Sciences, 16th and 18th eds., Mack Publishing, Easton PA (1980 & 1990).

The pH of a pharmaceutical composition or dosage form may also be adjusted to improve delivery of the active ingredient. Similarly, the polarity of a solvent carrier, its ionic strength, or tonicity can be adjusted to improve delivery. Compounds such as stearates can also be added to pharmaceutical compositions or dosage forms to advantageously alter the hydrophilicity or lipophilicity of one or more active ingredients so as to improve delivery. In this regard, stearates can serve as a lipid vehicle for the formulation, as an emulsifying agent or surfactant, and as a delivery-enhancing or penetration-enhancing agent. Different salts, hydrates or solvates of the active ingredients can be used to further adjust the properties of the resulting composition.

This disclosure encompasses kits which, when used by the medical practitioner, can simplify the administration of appropriate amounts of active ingredients to a patient.

A typical kit of the disclosure comprises a dosage form of the compound of the disclosure, or a tautomeric isoform, a pharmaceutically acceptable salt, solvate, hydrate, stereoisomer, prodrug, or clathrate thereof. Kits encompassed by this disclosure can further comprise additional active agents. Kits of the disclosure can further comprise devices that are used to administer the active ingredient. Examples of such devices include, but are not limited to, syringes, drip bags, patches, and inhalers. In an advantageous embodiment, a kit of the disclosure contains HK4.

Kits of the disclosure can further comprise cells or blood for transplantation as well as pharmaceutically acceptable vehicles that can be used to administer one or more active ingredients. For example, if an active ingredient is provided in a solid form that must be reconstituted for parenteral administration, the kit can comprise a sealed container of a suitable vehicle in which the active ingredient can be dissolved to form a particulate-free sterile solution that is suitable for parenteral administration. Examples of pharmaceutically acceptable vehicles include, but are not limited to: Water for Injection USP; aqueous vehicles such as, but not limited to, Sodium Chloride Injection, Ringer's Injection, Dextrose Injection, Dextrose and Sodium Chloride Injection, and Lactated Ringer's Injection; water-miscible vehicles such as, but not limited to, ethyl alcohol, polyethylene glycol, and polypropylene glycol; and non-aqueous vehicles such as, but not limited to, corn oil, cottonseed oil, peanut oil, sesame oil, ethyl oleate, isopropyl myristate, and benzyl benzoate.

Advantageous example for the compound according to the present disclosure for the use in the treatment of NAFLD and related diseases:
A) HK4 or 2-Cyano-N-(4-cyano-3-methyl-phenyl)-3-cyclopropyl-3-hydroxy-thioacrylamide

The following examples and methods are offered for illustrative purposes only and are not intended to limit the scope of the present disclosure in any way.

### Pharmacological data and activity

A series of non-clinical pharmacology and toxicology studies have been performed to support the clinical evaluation of the compound according to the present disclosure in human subjects. These studies were performed in accordance with internationally recognized guidelines for study design and in compliance with the requirements of Good Laboratory Practice (GLP) unless otherwise noted.

### Example I

Palmitate acid (PA) induced apoptosis and assessment of Caspase 3/7 activity in HepG2 cells.

### Method

NAFLD is characterized by excessive hepatic fat accumulation which leads to cell death, including apoptosis, of liver cells in the progression of the disease.

HepG2s (liver cell line) were seeded in a 96-well plate and cultured for 48 h before inducing cell death/apoptosis with 100 µM, 150 µM, 200 µM, 250 µM, 300 µM BSA-coupled palmitic acid (PA). A concentration-response curve to palmitate for apoptosis expressed as percentage of control of Caspase 3/7 (apoptosis marker) activity of HepG2 cells treated for 24 h were determined. As a positive control for apoptosis 2 µM staurosporine was used. Caspase 3/7 activity after 24 h was measured by a Caspase-Glo^{®} 3/7 Assay (Promega) as described in the manufacturer's protocol. Luminescence was recorded after an incubation step of 35 min using the plate-reader luminometer of a Cytation^{™} 5.

### Results:

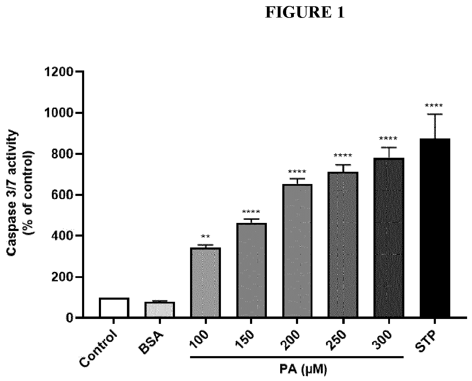

As shown in Figure 1: Palmitate acid (PA) significantly enhanced apoptosis upon a concentration of 100 µM (342.2 ± 12.25 %; p<0.01) For all other experiments a submaximal concentration of 200 µM was chosen to test the potential effect of HK4 on HepG2 cells.

### Example 2

HK4 prevents palmitate-induced apoptosis in hepatocytes (HepG2 cells) and potentiates GABA effect.

### Method

HepG2 cells were treated with palmitate acid (200 µM) in the presence or absence of HK4 (100 nM) and GABA (100 µM), as well as with the vehicle BSA and the positive control for apoptosis staurosporine (STP) (2 µM) for 24 h. As a negative control an apoptosis inhibitor (API) (100 µM) was used. As described in Example I Caspase 3/7 activity after 24 h was measured by a Caspase-Glo^{®} 3/7 Assay (Promega).

### Results:

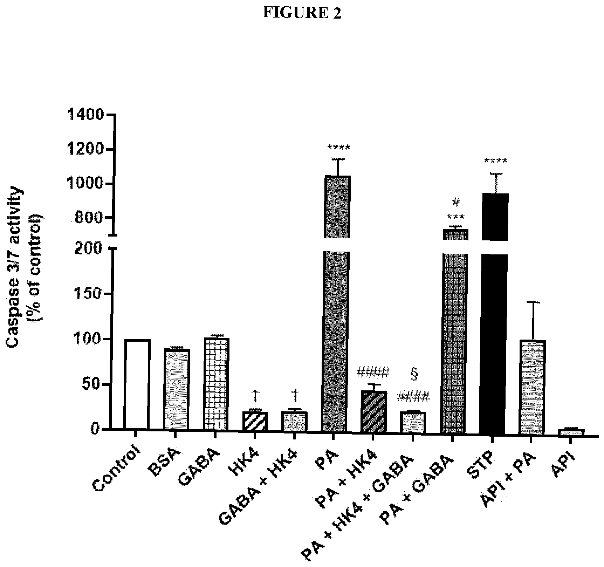

As shown in figure 2 HK4 alone (21.60 ± 2.52 %) reduced the caspase 3/7 activity compared to untreated control cells (p<0.0001). Additionally, HK4 was also able to completely prevent the caspase 3/7 activity provoked by palmitate 200 µM (47.32 ± 19.56 %, 1130 ± 103.3 % respectively, p<0.0001). Not only HK4, but also GABA slightly reduced the PA-induced apoptosis rate (764.9 ± 16.72 %, p<0.05), however not as potent as the positive allosteric modulator HK4. If GABA and its PAM HK4 were combined, the beneficial effect of HK4 on PA-induced apoptosis was even potentiated (24.43 ± 1.25 % and 47.32 ± 19.56 % respectively, p<0.05).

### Example 3

A representative human apoptosis array with marked spots for Caspase 7.

### Method

Markers of apoptosis were analysed in the cell lysates of treated HepG2s with a human apoptosis array (RayBiotech, Peachtree Corners, GA) according to manufacturer's protocol. The total protein content was measured using the Pierce BCA protein assay kit (Thermo Fisher Scientific). Membranes were incubated with 250 µg of total protein from untreated and treated HepG2 cells. The spot intensities were quantified after an exposure of 500 ms using the ChemStudio analysis software (Analytik Jena) and normalized to the untreated control after background subtraction with the design spots for it.

### Results:

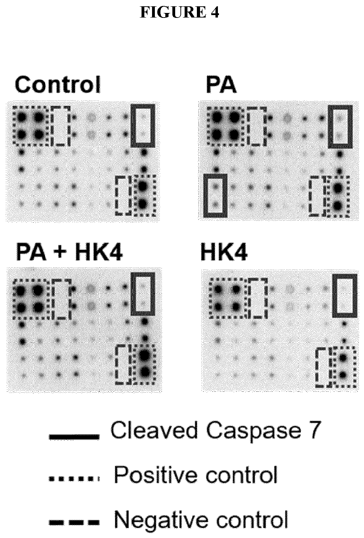

Caspase 7 protein expression was determined in human apoptosis arrays (Figure 4). The findings support the before mentioned increase of Caspase 7 activity during PA treatment and the preventive effect of HK4 (100 nM). In detail, Caspase 7 protein showed a significantly reduced expression in untreated cells (32.42 ± 18.57 %, p<0.05 vs. PA alone) and in cells treated with HK4 and PA (60.91 ± 4.18 %, p<0.001 vs. PA alone).

### Example 4

HK4 prevents palmitate-induced apoptosis in human primary hepatocytes.

### Method

Human primary hepatocytes were purchased from Thermo Fisher and cultured according to the manufacturer's instructions. Unless otherwise stated, cells were treated with 100 µM or 200 µM bovine serum albumin-conjugated palmitate alone or in combination with 100 nM HK4 1 h prior to palmitate exposure for 24 h. To achieve finale palmitate concentrations, sodium palmitate was complexed with a preheated BSA (Carl Roth, Karlsruhe, Germany) solution at a 2.6:1 molar ratio.

### Results:

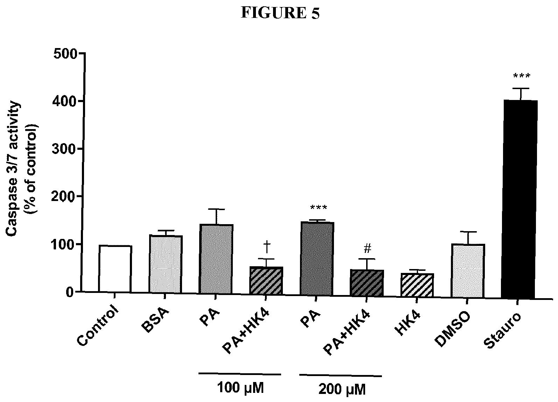

As shown in Figure 5 the preventive effect of HK4 on apoptosis has been confirmed in primary human hepatocytes. Pre-treatment with HK4 (10 µM) prevented caspase 3/7 activity provoked by palmitate 100 µM (58.73 ± 16.86 % and 147.3 ± 31.3 %, respectively, p<0.05) and by palmitate 200 µM (57.13 ± 21.47 % vs. 155.1 ± 3.78 %, p<0.05).

### Example 5

HK4 prevents the palmitate-induced increase in the protein ratio of phosphorylated NF-κB to the non-phosphorylated NF-κB subunit p65.

### Method

Beside excessive hepatic fat accumulation, inflammation is one of the critical steps in the progression of NAFL to NASH. As an indicator of inflammation, phosphorylation of the transcription factor NF-κB was monitored by a phospho-p65 specific antibody using protein extraction and western blotting.

HepG2 cells were homogenized in lysis buffer including a protease and phosphatase inhibitor cocktail. Cell lysates were centrifuged at 12.000 rpm for 15 min. The protein concentrations were determined using the Pierce BCA protein assay kit (Thermo Fisher Scientific). 20 µg of Proteins were resolved on 4-20% gradient gels (Bio-Rad, Hercules, CA) and transferred onto an Immun-Blot PVDF membrane (Bio-Rad). Membranes were blocked with 5 % non-fat dry milk or bovine serum albumin in TBPS containing 0.1 % Tween for 1 h at RT, followed by an overnight incubation with the respective primary antibody: Phospho-NF-κB p65 and NF-κB p65 (Cell Signaling Technology, Frankfurt, Germany). Membranes were incubated with the corresponding HRP-coupled secondary antibody. Protein bands were visualized using an ECL Western blot detection substrate (Bio-Rad). The band intensities were quantified with the ChemStudio analysis software (Analytik Jena, Jena, Germany) and normalized by GAPDH band intensity.

### Results:

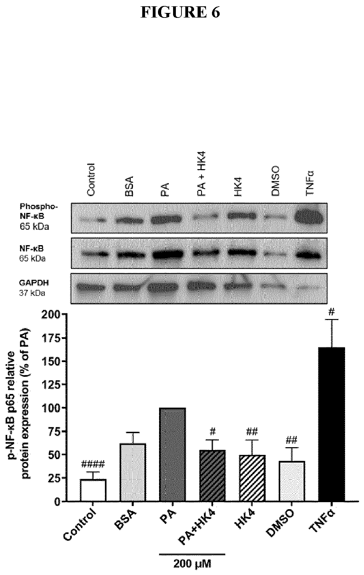

As shown in figure 6 palmitate acid led to an increased protein level of the ratio of the phosphorylated NF-κB compared to the non-phosphorylated NF-κB subunit p65. This effect was prevented by pre-incubation with HK4 (55.24 ± 10.64 % vs 100 % of PA, p<0.05). HK4 alone or its vehicle DMSO had no effect on the phosphorylation of the NF-κB subunit p65 compared to untreated cells.

### Statistical analysis of the experiments

Statistical analysis was performed using the GraphPad Prism software (Version 8.0.1, San Diego, USA). Unpaired two-tailed Student's t-test or one-way ANOVA (post hoc test: Bonferroni or Dunnett's) were used to determine statistical significance considering a p-value below 0.05 as statistically significant. Data are expressed as mean values ± SEM.

Altogether the data presented in these examples clearly demonstrate that HK4 treatment alone or in combination with GABA can prevent and reduce lipotoxic-induced apoptosis and inflammation in liver cells. HK4 arises as a potential innovative pharmacotherapy to treat or prevent NAFLD-associated hepatocyte injury.

The embodiments of the disclosure described above are intended to be merely exemplary, and those skilled in the art will recognize, or will be able to ascertain using no more than routine experimentation, numerous equivalents of specific compounds, materials, and procedures. All such equivalents are considered to be within the scope of the disclosure.

### Additional References

Norikura, T.; Kojima-Yuasa, A.; Opare Kennedy, D.; Matsui-Yuasa, I. Protective effect of gamma-aminobutyric acid (GABA) against cytotoxicity of ethanol in isolated rat hepatocytes involves modulations in cellular polyamine levels. Amino Acids 2007, 32, 419-423
Hata, T.; Rehman, F.; Hori, T.; Nguyen, J.H. GABA, γ-Aminobutyric Acid, Protects Against Severe Liver Injury. J. Surg. Res. 2019, 236, 172-183
Shilpa, J.; Roshni, B.T.; Chinthu, R; Paulose, C.S. Role of GABA and serotonin coupled chitosan nanoparticles in enhanced hepatocyte proliferation. J Mater Sci: Mater Med 2012, 23, 2913-2921
Wang, S.; Xiang, Y.-Y.; Zhu, J.; Yi, F.; Li, J.; Liu, C.; Lu, W.-Y. Protective roles of hepatic GABA signaling in acute liver injury of rats. Am. J. Physiol. Gastrointest. Liver Physiol. 2017, 312, G208-G218
The following additional publications are incorporated herein by references:
Raza, S., et al., 2021. Current treatment paradigms and emerging therapies for NAFLD/NASH. Front Biosci (Landmark Ed). 2021 Jan 1; 26:206-237.
Streba LAM, Vere CC, Rogoveanu I, Streba CT. Nonalcoholic fatty liver disease, metabolic risk factors, and hepatocellular carcinoma: an open question. World J Gastroenterol. 2015;21(14):4103-10.
Koopman KE, Caan MWA, Nederveen AJ, Pels A, Ackermans MT, Fliers E, et al. Hypercaloric diets with increased meal frequency, but not meal size, increase intrahepatic triglycerides: a randomized controlled trial. Hepatology. 2014;60(2):545-53.
Vilar-Gomez E, Martinez-Perez Y, Calzadilla-Bertot L, Torres-Gonzalez A, Gra-Oramas B, Gonzalez-Fabian L, et al. Weight loss through lifestyle modification significantly reduces features of nonalcoholic steatohepatitis. Gastroenterology. 2015; 149(2):367-378.e5.
Keating SE, Hackett DA, George J, Johnson NA. Exercise and non-alcoholic fatty liver disease: a systematic review and meta-analysis. J Hepatol. 2012;57(1): 157-66.
Kistler KD, Brunt EM, Clark JM, Diehl AM, Sallis JF, Schwimmer JB. Physical activity recommendations, exercise intensity, and histological severity of nonalcoholic fatty liver disease. Am J Gastroenterol. 2011;106(3):460-8 (quiz 469).
Pydyn N, Mi 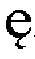 kus K, Jura J & Kotlinowski J. New therapeutic strategies in nonalcoholic fatty liver disease: a focus on promising drugs for nonalcoholic steatohepatitis. Pharmacological Reports volume 72, pages 1-12 (2020).
U.S: patent nos. 5,532,259,
WO 91/717748

## Claims

1. A compound for use in treating non-alcoholic fatty liver disease and related diseases, wherein the compound is 2-Cyano-N-(4-cyano-3-methyl-phenyl)-3-cyclopropyl-3 -hydroxy-thioacrylamide.

2. The compound according to claim 1, wherein the compound has the formula (I) 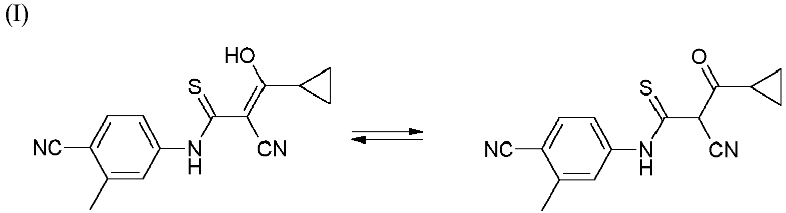 or tautomeric isoforms, or pharmaceutically acceptable salts, solvates or stereoisomers thereof.

3. The compound according to claim 2, wherein the stereoisomer of the compound is the enol or keto tautomer.

4. The compound according to claim 2, wherein the stereoisomer of the compound is the R or S enantiomer.

5. The compound of any of claims 1 to 4, wherein the non-alcoholic fatty liver disease is non-alcoholic fatty liver caused by excessive fat overload of the liver.

6. The compound of any of claims 1 to 4, wherein the non-alcoholic fatty liver disease is non-alcoholic steatohepatitis caused by excessive fat overload of the liver and inflammation of the liver.

7. A pharmaceutical composition for the use in the treatment of non-alcoholic fatty liver disease and related diseases comprising a compound as defined in claims 1 to 4 in free form or in the form of a tautomer or in the form of pharmaceutically acceptable salt or physiologically functional derivative, together with pharmaceutically acceptable diluents or carriers.

8. A pharmaceutical composition for preventing and/or treating of non-alcoholic fatty liver disease and related diseases, which comprises a therapeutically effective amount of 2-Cyano-N-(4-cyano-3-methyl-phenyl)-3-cyclopropyl-3-hydroxy-thioacrylamide or in the form of a tautomer or a physiologically functional derivative thereof in admixture with a pharmaceutical acceptable carrier or excipient.

9. A pharmaceutical composition for preventing and/or treating of non-alcoholic fatty liver disease and related diseases, which comprises a therapeutically effective amount of a compound as defined in claim 2 or a physiologically functional derivative thereof in admixture with a pharmaceutical acceptable carrier or excipient.

10. The compound for use according to any of the claims 1 to 6 or the composition according to any of claims 7 to 9 further comprising gamma-Aminobutyric-acid.

11. The compound for use according to any one of the claims 1 to 6 or the composition according to any of claims 7 to 9, wherein said compound is administered at daily dosages between 0,1 mg-10g/animal or human body, preferable 5 mg-5g/ animal or human body and more preferable 10 mg-2g/ animal or human body.
